# EUROPEAN PATENT SPECIFICATION

(11) **EP 2 853 208 B1**
(45) Date of publication and mention of the grant of the patent: **18.05.2022**
(21) Application number: 14184055.3
(22) Date of filing: 09.09.2014
(51) Int. Cl.: A61B 17/32, A61B 17/00

(54) **Ultrasonic dissector and sealer**
Ultraschalldissektor und -versiegeler
Dissecteur et scelleuse à ultrasons

(30) Priority: 25.09.2013 US 201361882035 P; 21.08.2014 US 201414464832
(43) Date of publication of application: 01.04.2015
(73) Proprietor: Covidien LP, Mansfield, MA 02048 (US)
(72) Inventor: Van Tol, David J., Boulder, Colorado 80301 (US); Ross, Anthony B., Boulder, Colorado 80301 (US); Stoddard, Robert B., Steamboat Springs, CO 80487 (US)
(74) Representative: Soames, Candida Jane

(56) References cited:
- WO-A1-93/14709
- WO-A1-2006/059120
- WO-A2-2004/026104
- US-A- 6 117 152
- US-A1- 2008 234 710
- US-A1- 2009 216 157
- US-B1- 6 454 781
- US-B1- 6 669 690

## Description

### Background

### 1. Technical Field

The present disclosure relates to surgical instruments and, more specifically, to ultrasonic surgical instruments having features for dissecting and sealing tissue.

### 2. Discussion of Related Art

Energy-based tissue treatment is well known in the art. Various types of energy (e.g., electrical, ultrasonic, microwave, cryogenic, thermal, laser, etc.) are applied to tissue to achieve a desired result. Ultrasonic energy may be delivered to tissue using a surgical probe that includes a transducer coupled with an end effector configured to deliver the ultrasonic energy to tissue.

A typical ultrasonic surgical instrument generates a sinusoidal driving signal that causes the mechanical tip of a waveguide to vibrate at a selected frequency, usually from about 20 KHz to about 60 KHz, for cutting and/or coagulating tissue. Improved cutting may result from increased tissue-to-mechanical tip contact caused by the high frequency of vibration of the mechanical tip in relation to tissue. Improved coagulation may result from heat generated by contact between the high frequency vibrations of the mechanical tip and body tissue.

Ultrasonic surgical instruments may include various waveguides configured to achieve a surgical result. An ultrasonic waveguide may be disposed at a distal end of the ultrasonic instrument. The waveguide may include an end effector having a cutting blade, shears, a hook, a ball, etc., and may also include other features such as jaws for grasping or manipulating tissue.

US2008234710, US6117152, US2009216157 and US6454781 describe examples of surgical instruments with an ultrasonic blade coupled to an ultrasonic transducer.

WO93/14709 describes an ultrasonic surgical blade for use in a surgical instrument. WO2006059120 describes an ultrasonic blade for use in surgery to bones.

WO2004026104 describes an ultrasonic surgical instrument including a jaw with a fluid management channel.

US 6 669 690 discloses features on which the preamble of the independent claim is based.

### Summary

According to the invention, there is provided a tool assembly as recited in the independent claim 1 with preferred features as set forth in the dependent claims. An aspect of the invention also provides an ultrasonic surgical instrument including a tool assembly of the invention.

In aspects of the present disclosure, the distal portion of the blade includes a planar distal tip.

The notch may include a vertical surface.

In aspects of the present disclosure, the bottom portion of the ultrasonic blade includes converging planar surfaces.

In aspects, the notch is located distally along the distal portion of the blade.

In aspects, the blade includes tapered segments tapering down between the side segments and a distal tip of the blade.

In aspects, the proximal portion is generally conical and tapers down to the distal portion.

In aspects, the distal portion includes an arcuate top portion and/or an arcuate bottom portion.

In some embodiments, the top and bottom portions have a common center of curvature.

In aspects, the top portion is configured to conform to a surface of clamp member contacting the top portion.

Also disclosed herein but not forming part of the invention is a method for ultrasonically treating tissue includes accessing a surgical site with an ultrasonic surgical instrument, dissecting tissue with a first portion of a blade, and sealing tissue with a second portion of the blade different that is different from the first portion of the blade. The instrument includes the blade that defines a longitudinal axis. The first portion may be a bottom portion of the blade. The second portion may be one of a top portion or planar side segments. Sealing the tissue may include clamping tissue between a top portion and a jaw pivotally movable relative to the blade.

Further, to the extent consistent, any of the aspects described herein may be used in conjunction with any or all of the other aspects described herein.

### Brief Description of the Drawings

Various aspects of the present disclosure are described herein below with reference to the drawings, wherein:
FIG. 1 is a perspective view of one illustrative example of a surgical instrument for use with the present disclosure;
FIG. 2 is an exploded view of components of an elongated body portion of the surgical instrument of FIG. 1;
FIG. 3 is an enlarged view of a tool assembly of the surgical instrument of FIG. 1 with a portion of an outer tube of the elongated body portion of FIG. 2 cut away;
FIG. 4 is an enlarged view of the distal end of the tool assembly of FIG. 3 in the closed position;
FIGS. 5A-B and 5D-G are side views of various examples of a blade of the surgical instrument of FIG. 1 not forming part of the present invention and FIG. 5C is an embodiment of a blade in accordance with the present invention; and
FIGS. 6A-G are front views of the blades of FIGS. 5A-G, respectively, with FIG. 6C being in accordance with the present invention.

### Detailed Description

Embodiments of the present disclosure are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to the portion of the device or component thereof that is closer to the clinician and the term "distal" refers to the portion of the device or component thereof that is further from the clinician.

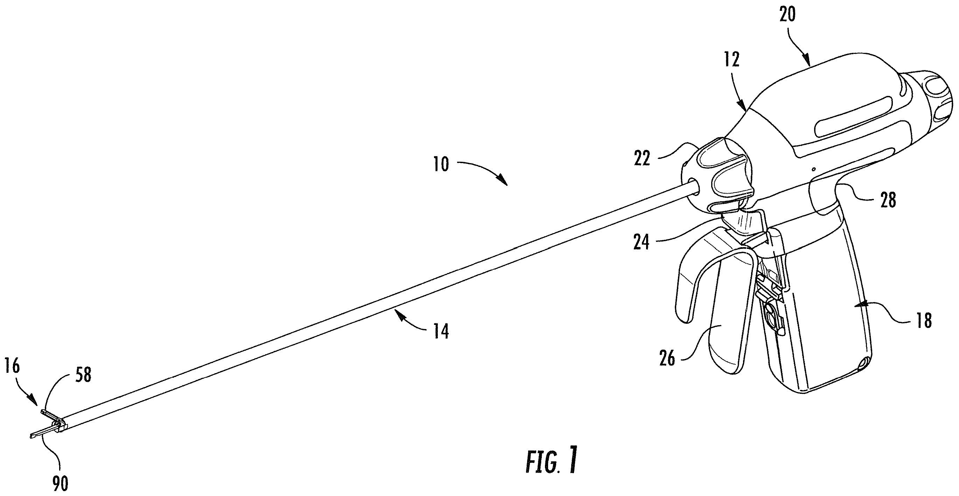

Referring now to FIG. 1, an ultrasonic surgical instrument 10 is shown. Ultrasonic surgical instrument 10 generally includes a handle assembly 12, an elongated body portion 14, and a tool assembly 16. Handle assembly 12 supports a battery assembly 18 and an ultrasonic transducer and generator assembly (hereinafter "TAG") 20. Handle assembly 12 includes a rotatable nozzle 22, an activation button 24, and a clamp trigger 26. Battery assembly 18 and TAG 20 are each releasably secured to a central body 28 of handle assembly 12 and are removable from central body 28 to facilitate disposal of the entire device, with the exception of battery assembly 18 and TAG 20.

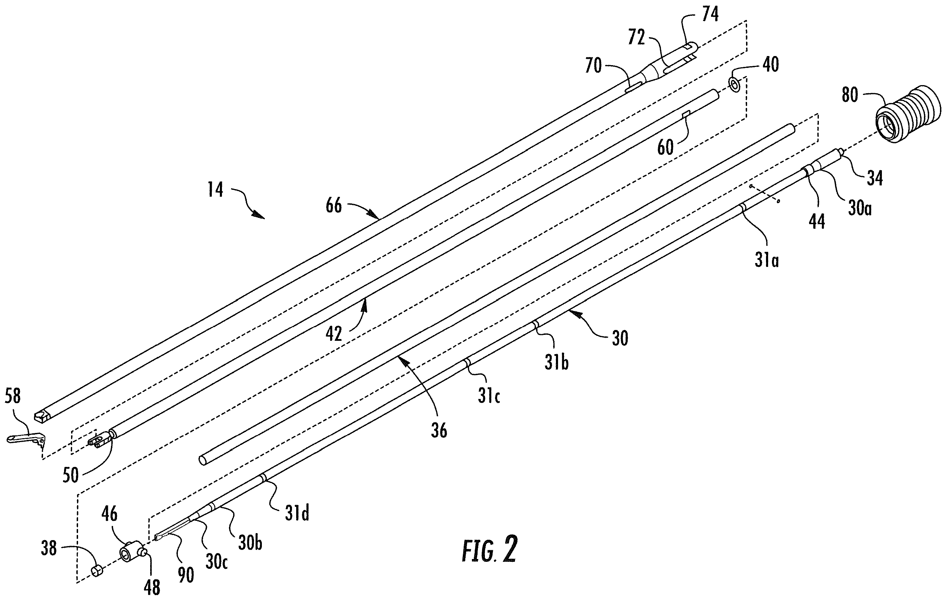

With additional reference to FIG. 2, elongated body portion 14 includes a waveguide 30 that extends from handle assembly 12 and couples tool assembly 16 (FIG. 1 ) thereto. A distal end of waveguide 30 defines a blade 90, which is described in further detail below. A proximal end of waveguide 30 has a threaded extension 34 for engaging TAG 20. Waveguide 30 further includes a proximal tapered portion 30a and distal tapered portions 30b and 30c. A series of annular abutments 31a-d are disposed along, e.g., machined onto, waveguide 30 at node points along waveguide 30.

An inner tube 36 is positioned about waveguide 30 between proximal tapered portion 30a and distal tapered portion 30b of waveguide 30. A distal seal member 38 is supported about waveguide 30 distally of a distal end of inner tube 36 and proximally of distal tapered portion 30c of waveguide 30 to provide a fluid-tight seal at the distal end of elongated body portion 14 between waveguide 30 and an inner surface of a middle tube 42. Ultrasonic energy is isolated from transfer to middle tube 42 by inner tube 36. A series of splines 44 are formed at the proximal end of waveguide 30. Splines 44 engage corresponding splines (not shown) formed on an inner surface of a torque adapter 46 to rotatably secure torque adapter 46 to waveguide 30. Torque adapter 46 also includes diametrically opposed wings 48 that are positioned in recesses (not shown) in rotatable nozzle 22 to secure torque adapter 46 to rotatable nozzle 22.

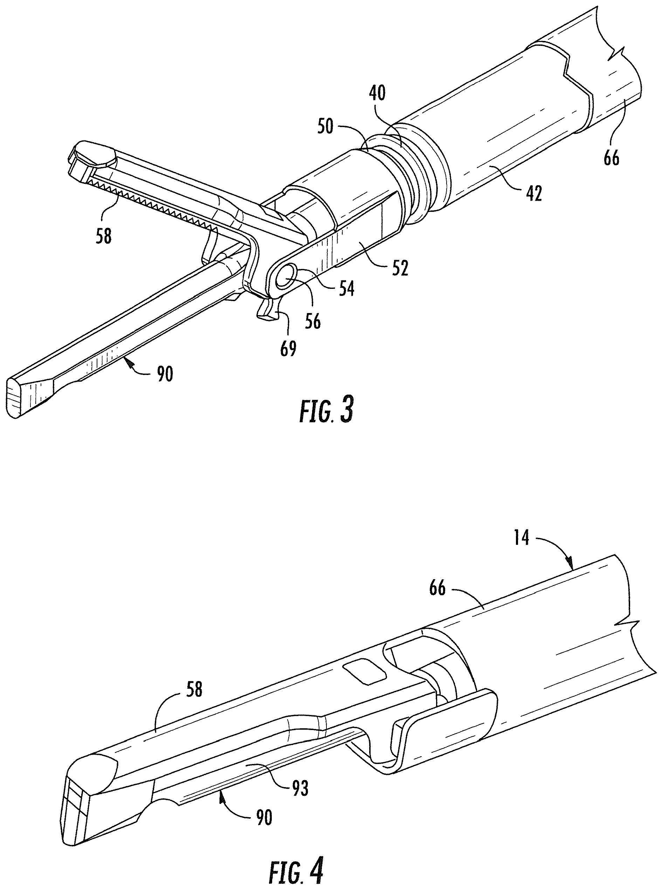

With additional reference to FIGS. 3 and 4, middle tube 42 is positioned about inner tube 36 (FIG. 2 ) and includes a distal end having a corset feature 50 and a pair of spaced clamp support arms 52. Corset feature 50 is positioned to receive distal seal member 38 and to maintain distal seal member 38 in the proper position about the distal end of waveguide 30. Distal seal member 38 is positioned at a node point along waveguide 30. An O-ring 40 is supported about corset feature 50 to provide a fluid-tight seal between an outer surface of middle tube 42 and an inner surface of an outer tube 66.

With reference to FIGS. 2-4, spaced clamp support arms 52 each define an opening 54 for pivotally receiving pivot members 56 formed on a clamp member 58 of tool assembly 16. Clamp member 58 of tool assembly 16 is pivotal between an open position (FIG. 3 ), wherein clamp member 58 is spaced from blade member 90, and a closed position ( FIG. 4 ), wherein clamp member 58 is in juxtaposed alignment with blade member 90. Clamp member 58 is moved between the open position and the closed position in response to actuation of clamp trigger 26 ( FIG. 1 ).

Referring again to FIG. 2, outer tube 66 is slidably repositionable between an advanced position and a retracted position. Upon movement of outer tube 66 from the advanced position to the retracted position, clamp member 58 is moved from the open position ( FIG. 3 ) to the closed position ( FIG. 4 ). A proximal end of outer tube 66 includes an elongated slot 70 ( FIG. 2 ) which receives projections (not shown) of rotatable nozzle 22 (FIG. 1) such that outer tube 66 is rotatably secured to, but slidable about, the projections to facilitate movement of outer tube 66 between the advanced and retracted positions.

The proximal end of outer tube 66 includes a bifurcated portion that defines an axially extending throughbore 72 that slidably receives wings 48 of torque adapter 46. A pair of diametrically opposed windows 74 are formed in the proximal end of outer tube 66. Windows 74 receive bosses (not shown) formed in handle assembly 12 (FIG. 1) to couple outer tube 66 to handle assembly 12 (FIG. 1).

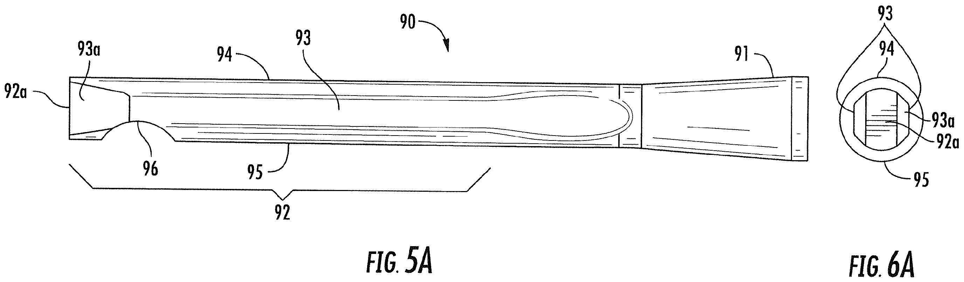

With reference to FIGS 5A and 6A, blade 90 defines a longitudinal axis and includes a proximal portion 91 and a distal portion 92. Proximal portion 91 is configured to couple to waveguide 30. In embodiments, proximal portion 91 may be generally conical and may taper down to distal portion 92. Distal portion 92 includes a planar distal tip 92a, planar side segments 93, an arcuate top portion 94, an arcuate bottom portion 95, and a notch 96. Side segments 93 are substantially flat parallel to the longitudinal axis of blade 90. Side segments 93 include flat tapered segments 93a near distal tip 92a such that tapered segments 93a form a flat surface at distal tip 92a. Side segments 93 and/or tapered segments 93a may be configured to score or seal tissue as described in detail below. In embodiments, top portion 94 may be generally arcuate. Notch 96 is disposed on bottom portion 95 and is configured to cut or dissect tissue as described in detail below. Top portion 94 contacts clamp member 58 when clamp member 58 is in the closed position. In some embodiments, top portion 94 may be configured to conform to the surface of clamp member 58.

In use, blade 90 may be used to dissect and seal tissue. During operation, TAG 20 is activated to ultrasonically translate or oscillate blade 90 along the longitudinal axis. As blade 90 oscillates, the temperature of blade 90 increases. When the temperature of blade 90 reaches a desired temperature, distal portion 92 of blade 90 may be used to dissect and/or seal tissue. The cutting properties of distal portion 92 are improved as the temperature of distal portion 92 increases. It is envisioned that any portion of distal portion 92 may be used to dissect tissue. In embodiments, notch 96 of distal portion 92 may be used to improve cutting properties of distal portion 92. Notch 92 may increase tissue contact or tissue tension during a cutting or back scoring motion to improve the cutting properties of bottom portion 95. In some embodiments, clamp member 58 may be used to increase the tension in tissue clamped between clamp member 58 and top portion 94 of blade 90.

When the temperature of blade 90 is increased, as described above, planar surfaces, e.g., side segments 93 of distal portion 92 of blade 90 may also be used to score or seal tissue. In embodiments, side segments 93 may seal tissue when the temperature of distal portion 92 is at or above 250° C. However, it is also envisioned that side segments 93 may seal tissue at temperatures below 250° C by applying additional pressure to the tissue to be sealed against side segments 93, e.g., it has been shown that tissue may be sealed at a temperature as low as 90° C. Top portion 94 of blade 90 may be used to seal tissue. Clamp member 58 may apply additional pressure to the tissue to be sealed against top portion 94.

The ultrasonic motion of blade 90 results in decreasing internal stress from the proximal portion 91 to distal tip 92a. Thus, location of notch 96 is based on reducing physical strain and/or stress on the blade 90. In embodiments, if notch 96 is disposed too proximally, the reduction in the cross-sectional area as well as the stress concentration of notch 96 may result in excessive stress causing fracture of blade 90.

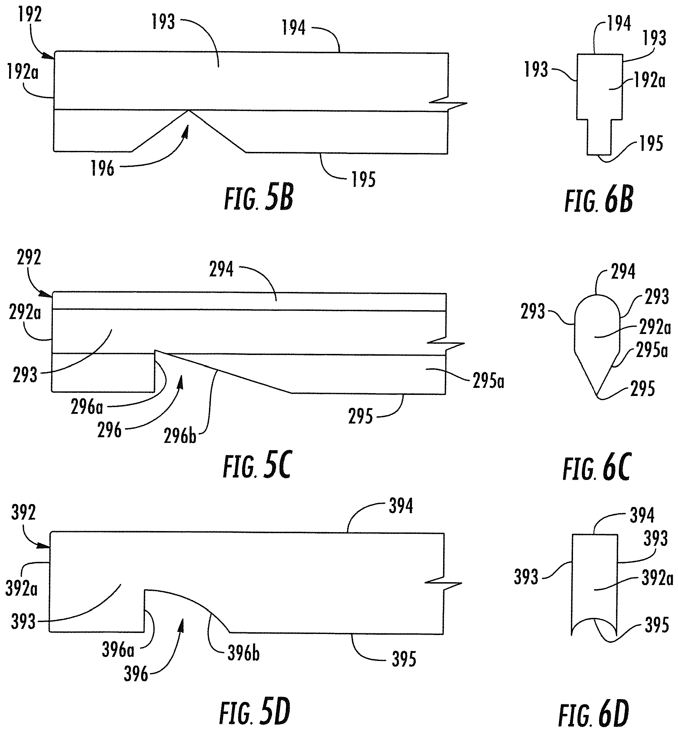

Referring to FIGS. 5B and 6B, an example of a distal portion 192 not forming part of the invention is shown. Distal portion 192 includes a planar distal tip 192a, planar side segments 193, a planar top portion 194, a planar bottom portion 195, and a triangular notch 196 disposed on bottom portion 195. Distal portion 192 is similar to distal portion 92 described above, as such only the differences are described in detail below for reasons of brevity. Bottom portion 195 has a stepped configuration as shown in FIG. 6B such that the bottom planar surface of bottom portion 195 is smaller than the planar surface of top portion 194, e.g., in a plane parallel to the longitudinal axis. Tip 192a, side segments 193, top portion 194, and bottom portion 195 are configured to seal tissue as described above. In addition, tip 192a, side segments 193, top portion 194, bottom portion 195, and notch 196 are configured to dissect tissue as described above.

Referring to FIGS. 5C and 6C, an embodiment of a distal portion 292 in accordance with the present invention is shown. Distal portion 292 includes a planar distal tip 292a, planar side segments 293, an arcuate top portion 294, a pointed bottom portion 295, and a notch 296. Distal portion 292 is similar to distal portion 92 described above, as such only the differences will be described in detail below for reasons of brevity. Bottom portion 295 includes planar surfaces 295a that converge to form a pointed edge. In some embodiments, the pointed edge at the convergence of planar surfaces 295a is sharpened to improve the cutting properties of bottom portion 295a. Notch 296 includes a vertical distal surface 296a and an angled planar surface 296b. Vertical distal surface 296a of notch 296 may also be sharpened to improve the cutting properties of notch 296. In this embodiment, tip 292a, side segments 293, top portion 294, and planar surfaces 295a of bottom portion 295 are configured to seal tissue as described above. In addition, tip 292a, side segments 293, top portion 294, bottom portion 295, and notch 296 are configured to dissect tissue as described above.

Referring to FIGS. 5D and 6D, still another example of a distal portion 392 not forming part of the invention is shown. Distal portion 392 includes a planar distal tip 392a, planar side segments 393, a planar top portion 394, a concave bottom portion 395, and a notch 396. Distal portion 392 is similar to distal portion 92 described above, as such only the differences will be described in detail below for reasons of brevity. Concave bottom portion 395 may be generally arcuate, which improves the cutting properties of distal portion 392 along bottom portion 395 between side segments 393. Notch 396 includes a vertical distal surface 396a and an arcuate surface 396b extending proximally therefrom. Vertical distal surface 396a may be sharpened to improve the cutting properties of notch 396. Tip 392a, side segments 393, and top portion 394 are configured to seal tissue as described above. In addition, tip 392a, side segments 393, top portion 394, bottom portion 395, and notch 396 are configured to dissect tissue as described above.

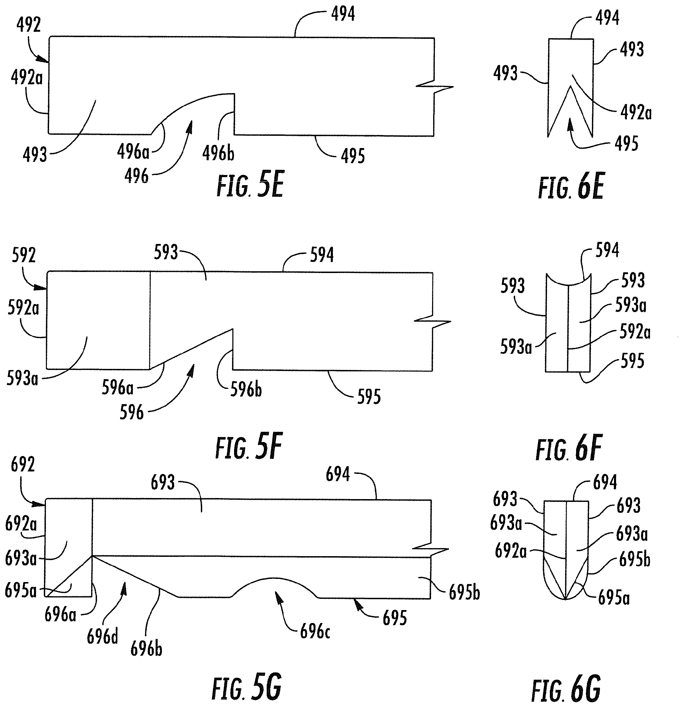

Referring to FIGS. 5E and 6E, still yet another example of a distal portion 492 not forming part of the invention is shown. Distal portion 492 includes a planar distal tip 492a, planar side segments 493, a planar top portion 494, a recessed bottom portion 495, and a notch 496. Distal portion 492 is similar to distal portion 92 described above, as such only the differences will be described in detail below for reasons of brevity. Recessed bottom portion 495 includes inwardly converging angled planar surfaces. The recess of bottom portion 495 may improve the cutting properties of distal portion 492 along bottom portion 495 between side segments 493. Notch 496 includes an arcuate surface 496a and a vertical proximal surface 496b. Vertical proximal surface 496b may be sharpened to improve the cutting properties of notch 496. Tip 492a, side segments 493, and top portion 494 are configured to seal tissue as described above. In addition, tip 492a, side segments 493, top portion 494, bottom portion 495, and notch 496 are configured to dissect tissue as described above.

Referring to FIGS. 5F and 6F, another example of a distal portion 592 not forming part of the invention is shown. Distal portion 592 includes a pointed distal tip 592a, planar side segments 593, a concave top portion 594, a planar bottom portion 595, and a notch 596. Distal portion 592 is similar to distal portion 92 described above, as such only the differences will be described in detail below for reasons of brevity. Pointed distal tip 592a may be sharpened to improve the cutting properties of distal tip 592a. Concave top portion 594 is generally arcuate, which improves the cutting properties of distal portion 592 along top portion 594 between side segments 593. Notch 596 includes an angled surface 596a and a vertical proximal surface 596b. Vertical proximal surface 596b may be sharpened to improve the cutting properties of notch 596. Side segments 593, tapered segments 593a, and bottom portion 595 are configured to seal tissue as described above. In addition, tip 592a, side segments 593, tapered segments, 593a, top portion 594, bottom portion 595, and notch 596 are configured to dissect tissue as described above.

Referring to FIGS. 5G and 6G, yet another example of a distal portion 692 not forming part of the invention is shown. Distal portion 692 includes a pointed distal tip 692a, planar side segments 693, a planar top portion 694, a bottom portion 695, a proximal notch 696c, and a distal notch 696d. Distal portion 692 is similar to distal portion 92 described above, as such only the differences will be described in detail below for reasons of brevity. Pointed distal tip 692a may be sharpened to improve the cutting properties thereof.

As shown in FIG. 6G, bottom portion 695 includes planar surfaces 695a and an arcuate surface 695b. Planar surfaces 695a converge to form a pointed tip. The pointed tip at the convergence of planar surfaces 695a may be sharpened to improve the cutting properties of a portion of bottom portion 695. Arcuate surface 695b is positioned proximal to planar surfaces 695a. Arcuate surface 695b may be positioned proximal to planar surfaces 695a. Proximal notch 696c is generally arcuate and distal notch 696d includes a vertical distal surface 696a and an angled planar surface 696b. Vertical distal surface 696a may be sharpened to improve the cutting properties of distal notch 696d. It is envisioned that any of the notches described above may be used in place of either of notches 696c, 696d. Side segments 693, tapered segments 693a, top portion 694, and planar surfaces 695a of bottom portion 695 may be configured to seal tissue as described above. In addition, tip 692a, side segments 693, tapered segments, 693a, top portion 694, bottom portion 695, and notches 696c, 696d are configured to dissect tissue as described above.

Distal portions 192, 292, 392, 492, 592, and 692 are used in a manner substantially similar to distal portion 92 described above, as such the use of these distal portions will not be discussed in detail.

Although various embodiments of distal portions have been described in detail above, it is envisioned that a distal portion may include any combination of features as described above. It is also envisioned that a distal portion may include more than two notches. Moreover, while particular elements of distal portions have been described as configured to seal and/or dissecting tissue, this is not meant to be limiting to the capabilities of these or other elements described above.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. The invention is defined by the appended claims.

## Claims

1. A tool assembly (10) comprising:
an ultrasonic blade (90) including a proximal portion and a distal portion extending from the proximal portion, the distal portion defining a longitudinal axis and **characterised by**:
an arcuate top portion (294) configured to seal tissue;
planar side segments (293) extending between the top and bottom portions; and
a bottom portion (295) including converging planar surfaces (295a) offset from each of the planar side segments (293) and at least one notch (296) disposed along a narrow edge formed by the converging planar surfaces, the notch (296) including a vertical distal surface (296a) and angled planar surface (296b) wherein at least one of the top portion, the bottom portion, or the at least one notch is configured to dissect tissue; and
a jaw member (58) configured to move relative to the ultrasonic blade and to contact the top portion.

2. The tool assembly of claim 1, wherein the distal portion of the ultrasonic blade includes a planar distal tip (292a).

3. An ultrasonic surgical instrument (10), comprising:
a handle assembly (12);
an elongated body (14) extending distally from the handle assembly, the elongated body defining a longitudinal axis and including a waveguide (30); and
a tool assembly (16) according to one of claims 1 to 2.

## Patentansprüche

1. Werkzeuganordnung (10), die Folgendes umfasst:
eine Ultraschallklinge (90), die einen proximalen Abschnitt und einen distalen Abschnitt beinhaltet, der sich aus dem proximalen Abschnitt erstreckt, wobei der distale Abschnitt eine Längsachse definiert und durch Folgendes gekennzeichnet ist:
einen bogenförmigen oberen Abschnitt (294), der konfiguriert ist, um Gewebe zu versiegeln;
ebene Seitensegmente (293), die sich zwischen dem oberen und dem unteren Abschnitt erstrecken; und
einen unteren Abschnitt (295), der konvergierende ebene Oberflächen (295a), die von jedem der ebenen Seitensegmente (293) versetzt sind, und wenigstens eine Kerbe (296) beinhaltet, die entlang einer schmalen Kante eingerichtet ist, die durch die konvergierenden ebenen Oberflächen ausgebildet wird, wobei die Kerbe (296) eine vertikale distale Oberfläche (296a) und eine abgewinkelte ebene Oberfläche (296b) beinhaltet, wobei der obere Abschnitt, der untere Abschnitt und/oder die wenigstens eine Kerbe konfiguriert ist, um Gewebe zu sezieren; und
ein Backenelement (58), das konfiguriert ist, um sich relativ zu der Ultraschallklinge zu bewegen und um den oberen Abschnitt zu berühren.

2. Werkzeuganordnung nach Anspruch 1, wobei der distale Abschnitt der Ultraschallklinge eine ebene distale Spitze (292a) beinhaltet.

3. Chirurgisches Ultraschallinstrument (10), das Folgendes umfasst:
eine Griffanordnung (12);
einen länglichen Körper (14), der sich aus der Griffanordnung distal erstreckt, wobei der längliche Körper eine Längsachse definiert und einen Wellenleiter (30) beinhaltet; und
eine Werkzeuganordnung (16) nach einem der Ansprüche 1 bis 2.

## Revendications

1. Ensemble outil (10) comprenant :
une lame ultrasonique (90) comportant une partie proximale et une partie distale s'étendant à partir de la partie proximale, la partie distale définissant un axe longitudinal et **caractérisée par** :
une partie supérieure arquée (294) conçue pour sceller du tissu ;
des segments latéraux plans (293) s'étendant entre les parties supérieure et inférieure ; et
une partie inférieure (295) comportant des surfaces planes convergentes (295a) décalées par rapport à chacun des segments latéraux plans (293) et au moins une encoche (296) disposée le long d'un bord étroit formé par les surfaces planes convergentes, l'encoche (296) comportant une surface distale verticale (296a) et une surface plane inclinée (296b) dans laquelle la partie supérieure et/ou la partie inférieure et/ou l'au moins une encoche est conçue pour disséquer du tissu ; et
un élément de mâchoire (58) conçu pour se déplacer par rapport à la lame ultrasonique et pour entrer en contact avec la partie supérieure.

2. Ensemble d'outils selon la revendication 1, dans lequel la partie distale de la lame ultrasonique comporte une pointe distale plane (292a).

3. Instrument chirurgical ultrasonique (10), comprenant :
un ensemble poignée (12) ;
un corps allongé (14) s'étendant de manière distale à partir de l'ensemble poignée, le corps allongé définissant un axe longitudinal et comportant un guide d'ondes (30) ; et
un ensemble outils (16) selon l'une des revendications 1 à 2.
